(19) Europäisches Patentamt
European Patent Office
Office européen des brevets

(11) **EP 1 755 702 B1**

(12) **EUROPÄISCHE PATENTSCHRIFT**

(45) Veröffentlichungstag und Bekanntmachung des Hinweises auf die Patenterteilung:
**12.12.2012 Patentblatt 2012/50**

(51) Int Cl.:
***A61M 1/10*** *(2006.01)*

(21) Anmeldenummer: **05750183.5**

(86) Internationale Anmeldenummer:
**PCT/AT2005/000203**

(22) Anmeldetag: **07.06.2005**

(87) Internationale Veröffentlichungsnummer:
**WO 2005/120601 (22.12.2005 Gazette 2005/51)**

(54) **VORRICHTUNG ZUR INTERMITTIERENDEN OKKLUSION DES KORONARSINUS**

DEVICE FOR THE INTERMITTENT OCCLUSION OF THE CORONARY SINUS

SYSTEME D'OCCLUSION INTERMITTENTE DU SINUS CORONAIRE

(84) Benannte Vertragsstaaten:
**CH DE GB LI**

(30) Priorität: **14.06.2004 AT 10122004**

(43) Veröffentlichungstag der Anmeldung:
**28.02.2007 Patentblatt 2007/09**

(73) Patentinhaber: **MIRACOR Medical Systems GmbH**
**1060 Wien (AT)**

(72) Erfinder: **Mohl, Werner**
**2571 Altenmarkt/Thennenberg (AT)**

(74) Vertreter: **Haffner, Thomas M.**
**Haffner und Keschmann**
**Patentanwälte KG**
**Schottengasse 3a**
**1014 Wien (AT)**

(56) Entgegenhaltungen:
**EP-A- 0 230 996    WO-A-03/008018**

**Beschreibung**

Die Erfindung betrifft eine Vorrichtung zur intermittierenden Okklusion des Koronarsinus, umfassend eine Okklusions-Einrichtung zum abwechselnden Okkludieren des Koronarsinus und Aufheben der Okklusion und eine Steuerungseinrichtung zur Abgabe von Steuersignalen an die Okklusions-Einrichtung zur Steuerung des Zeitpunkts des Beginns und des Aufhebens der Okklusion.

Arterielles Blut, das den Herzmuskel versorgt, kann durch gesundes Herzgewebe hindurchtreten und es ernähren, hat jedoch Schwierigkeiten das ischämische Gewebe zu erreichen. Dadurch wird die Versorgung des ischämischen Gewebes mit Nährstoffen sowie das Abführen von Abbauprodukten des Metabolismus von dem ischämischen Gewebe behindert.

In diesem zusammenhang ist bereits vorgeschlagen worden das ischämische Gewebe durch retrograde Perfusion mit Blut zu versorgen. Dabei wird versucht das Blut in Gegenrichtung von dem Koronarsinus zurück durch das koronare Venensystem fließen zu lassen, in dem man aus einer anderen Quelle Blut in den Koronarsinus einspeist, entweder durch permanente Verbindung einer Arterie mit dem Koronarsinus oder durch temporäres Einsetzen eines Katheters in den Sinus, der mit von einer entfernten Arterie entnommenem und mit Hilfe einer sich außerhalb des Körpers des Patienten befindlichen Blutpumpe beförderten Blut versorgt wird.

Bei einer anderen vorgeschlagenen Technik für die Retroperfusion wird ein am Ende eines Katheters festgelegter aufblasbarer Ballon verwendet, um den Koronarsinus intermittierend zu okkludieren. Der Blutdruck im Koronarsinus steigt während der Okklusion bei jedem Herzschlag, sodass Blut, das durch das gesunde Gewebe des Herzmuskels in den Koronarsinus gelangt, in das ischämische Gewebe zurückgeschwemmt wird. Bei einer solchen intermittierenden Koronarsinusokklusion wird das Ballonende des Katheters perkutan oder durch eine Operation eingesetzt. Das andere Ende des Katheters wird mit Gas oder Flüssigkeit durch eine Pumpe versorgt, die ein zyklisches Aufblasen und Zusammenfallen des Ballons bewirkt.

Ein typisches Anwendungsgebiet für die Retroinfusion von Blut in Koronarvenen mittels intermittierender Koronarsinusokklusion betrifft die Myokardprotektion während eines kurzfristigen Koronararterionverschlusses im Rahmen eines kardiologischen Eingriffes. Ein typischer derartiger Eingriff ist beispielsweise die Ballondilatation einer arteriosklerotisch verengten Koronararterie. Bei dieser auch als perkutane transluminale Koronarangioplastie (PTCA) bekannten Methode wird ein Ballonkatheter unter Röntgenkontrolle in den Bereich der Stenose der Koronararterie geführt und die arteriosklerotische Plaque durch Aufblasen des am Ende des Katheters befindlichen Ballons komprimiert. Während der Dilatation des Ballons findet stromabwärts in der Arterie keine Versorgung des Gewebes mit sauer-stoffhaltigem Blut statt, wobei sich bereits bei Dilatationen von länger als 30 Sekunden Dauer funktionelle Veränderungen im Ischämiegebiet des Myokards feststellen lassen. Entsprechende Probleme der Ischämieprotektion des Myokards stellen sich auch bei anderen Eingriffen zur Koronarvaskularisierung wie z.B. bei Atherektomie, Koronarendoprothesen, Laseranwendungen und perkutanen Herzklappenoperationen.

Eine Vorrichtung zur Retroinfusion von Koronarvenen ist beispielsweise aus der EP 230 996 A2 bekanntgeworden, mit welcher eine druckgesteuerte intermittierende Koronarsinusokklusion vorgenommen werden kann. Die Vorrichtung umfasst eine Einrichtung zum Okkludieren des Sinus wie z.B. einen aufblasbaren Ballonkatheter, eine Druckmesseinrichtung zum Messen des Flüssigkeitsdruckes innerhalb des Koronarsinus und ein Steuergerät, das Auslösesignale für die Okklusions-Einrichtung erzeugt, um eine Okklusion auszulösen oder aufzuheben. Das Steuergerät ist hiebei derart ausgelegt, dass im Koronarsinus das Druckmaximum während jedes Herzschlages gemessen, ein Plateauwert der Druckmaxima aufeinanderfolgender Herzschläge rechnerisch abgeschätzt und die Okklusion des Koronarsinus auf Basis des Plateauwertes der Druckmaxima aufgehoben wird.

Das Okkludieren des Koronarsinus bewirkt einen Druckanstieg und in der Folge eine Retroperfusion von Blut über die entsprechende Vene in die nutritiven Kapillaren des Ischämiegebietes, sodass diese Gebiete mit Nährstoffen versorgt werden können. Bei Aufheben der Okklusion wird das retroperfundierte Blut ausgeschwemmt, wobei gleichzeitig die Abfallprodukte des Metabolismus abgeführt werden. Bei dem Verfahren gemäß der EP 230 996 A2 wird somit aufgrund der Messung des Druckmaximums im Koronarsinus während jedes Herzschlages rechnerisch eine systolische Druckkurve abgeschätzt, wobei die Okklusion des Koronarsinus in Abhängigkeit von dem Plateauwert der systolischen Druckkurve aufgehoben wird.

Zur Bestimmung des Zeitpunkts, zu welchem die Okklusion wieder eingeleitet wird, wird gemäß dem Verfahren der EP 230 996 A2 eine empirisch gewonnene Formel vorgeschlagen, mit welcher der Intervall zwischen dem Ende der Okklusion und dem Spitzenwert der reaktiven Hyperämie abgeschätzt werden kann. Die empirisch gewonnene Formel basiert auf Parametern der für den okkludierten Koronarsinus bestimmten systolischen und diastolischen Druckkurve, wobei in der Folge eine lineare Regression vorgenommen wird. Mit dem verfahren gemäß der EP 230 996 A2 wurde zwar erreicht, dass die Okklusion erst zu einem Zeitpunkt wieder eingeleitet wird, welcher nach dem Auftreten des Spitzenwertes der reaktiven Hyperämie, d.h. nach dem Ausströmen des aufgestauten Blutes aus dem Koronarsinus, liegt. Die Ungenauigkeit der empirisch bestimmten Formel hat jedoch zur Folge, dass mitunter aus Sicherheitsgründen der Zeitraum bis zur erneuten Okklusion des Koronarsinus zu groß gewählt wird und somit zu lange zugewartet wird bis die nächste Okklusion eingeleitet wird. Dadurch wird das Verfahren ineffizient, wobei sich zu allem Überfluss herausgestellt hat, dass die rechnerische Durchführung des Verfahrens umständlich und nicht mit hinreichender Geschwindigkeit gelingt.

Die vorliegende Erfindung zielt nun darauf ab, eine Vorrichtung für die intermittierende Okklusion des Koronarsinus vorzuschlagen, mit welcher eine höhere Genauigkeit der Bestimmung des optimalen Zeitpunkts der Einleitung der nächsten Okklusion erreicht wird und welche unmittelbar auf die Druckverhältnisse im nicht okkludierten Koronarsinus Rücksicht nimmt.

Zur Lösung dieser Aufgabe zeichnet sich die erfindungsgemäße Vorrichtung dadurch aus, dass die mit der Steuerungseinrichtung Verbundene Recheneinheit zur rechnerischen Abschätzung des Verlaufs des nach Aufhebung der Okklusion im Koronarsinus auftretenden Flüssigkeitsdrucks ausgebildet ist, wobei der Zeitpunkt des Beginns der nächsten Okklusion in Abhängigkeit vom geschätzten Druckverlauf bestimmt wird. Erfindungsgemäß werden somit zur Bestimmung des Zeitpunkts des Beginns der nächsten Okklusion unmittelbar die Druckverhältnisse im Koronarsinus während der nicht okkludierten Phase (Releasephase) abgeschätzt und es ist somit nicht mehr erforderlich auf empirisch gewonnene Formeln zurückzugreifen, sodass die Genauigkeit des Verfahrens erhöht wird. Die Kenntnis bzw. die Vorhersage des Verlaufs des während der Releasephase im Koronarsinus herrschenden Drucks erlaubt unmittelbare Rückschlüsse auf verschiedene andere für das Verfahren entscheidende Parameter, wie beispielsweise das Durchflussvolumen und den Spitzenwert der reaktiven Hyperämie, und erlaubt die präzise Steuerung der Okklusions-Einrichtung.

Eine Verfeinerung des Verfahrens ergibt sich, wenn die nach Aufhebung der Okklusion im Koronarsinus während aufeinanderfolgender Herzschläge auftretenden Druckmaxima rechnerisch abgeschätzt werden und der Zeitpunkt des Beginns der nächsten Okklusion in Abhängigkeit von den geschätzten Druckmaxima bestimmt wird. Ähnlich kann auf vorgegangen werden, wenn anstatt der Druckmaxima die Druckminima herangezogen werden. Die erfindungsgemäße Vorrichtung ist in diesem Zusammenhang bevorzugt derart weitergebildet, dass die Recheneinheit zur Abschätzung der nach Aufhebung der Okklusion im Koronarsinus während aufeinanderfolgender Herzschläge auftretenden Druckmaxima ausgebildet ist, wobei der Zeitpunkt des Beginns der nächsten Okklusion in Abhängigkeit von den geschätzten Druckmaxima bestimmt wird.

Nach Aufhebung der Okklusion findet im Koronarsinus ein Relaxationsprozess statt, wobei der Druckverlauf durch eine Exponentialfunktion angenähert werden kann. Dabei kann derart vorgegangen werden, dass die nach Aufhebung der Okklusion im Koronarsinus während aufeinanderfolgender Herzschläge auftretenden Druckmaxima durch eine Exponentialfunktion angenähert werden und der Zeitpunkt des Beginns der nächsten Okklusion in Abhängigkeit vom Plateauwert der Exponentialfunktion bestimmt wird. Die erfindungsgemäße Vorrichtung ist hierbei derart weitergebildet, dass die Recheneinheit zur Approximation der nach Aufhebung der Okklusion im Koronarsinus während aufeinanderfolgender Herzschläge auftretenden Druckmaxima durch eine Exponentialfunktion ausgebildet ist, wobei der Zeitpunkt des Beginns der nächsten Okklusion in Abhängigkeit vom Plateauwert der Exponentialfunktion bestimmt wird. Dabei kann wiederum in analoger Weise bei der Annäherung der Druckminima vorgegangen werden. Die Exponentialfunktion, welche die Druckmaxima bzw. Druckminima verbindet und daher die systolische bzw. diastolische Druckkurve wiedergibt, nähert sich asymptotisch einem Plateauwert, welcher dem niedrigsten Druck im Koronarsinus nach der Aufhebung der Okklusion bzw. dem Ausgangsdruck im Koronarsinus vor Einleitung der intermittierenden Okklusion entspricht. Dieser Plateauwert eignet sich daher besonders gut für die Bestimmung der Okklusionsintervalle. Es kann dabei bevorzugt beispielsweise derart vorgegangen werden, dass die Okklusion des Koronarsinus aufgehoben wird, wenn die Exponentialfunktion ein definiertes Vielfaches des prognostizierten Plateauwertes erreicht. In diesem Fall wird die Releasephase dann beendet, wenn der Druck im Koronarsinus gemäß dem prognostizierten exponentiellen verlauf einen gewissen Prozentsatz über dem Plateauwert liegt. Die Exponentialfunktion kann hierbei besonders bevorzugt definiert werden durch:

$$p(t) = a + b \bullet e^{-t/c},$$

wobei

a    der systolische bzw. diastolische Druck im Koronarsinus vor der intermittierenden Okklusion,

b    die Differenz zwischen dem systolischen bzw. diastolischen Druck am Ende der Okklusion und dem Druck a,

c    eine Zeitkonstante

darstellen. Dabei sind lediglich die drei Parameter a, b und c zu bestimmen, wobei zwei der drei Parameter, nämlich die Parameter a und b, messbare Größen sind, welche während des Eingriffes ermittelt werden können. C entspricht hierbei einer Zeitkonstante, welche der Zeit entspricht, in welcher der Koronarsinusdruck auf 1/e - 36,8% seines maximalen Werts abfällt. Dadurch, dass zwei der drei Parameter messbaren Größen darstellen, welche individuell für jeden Patienten bestimmt werden können, wird mit der oben genannten Exponentialfunktion ein Verfahren geschaffen, welches besonders stabil ist und reproduzierbare werte für die Okklusionsintervalle ergibt. Die Zeitkonstante c kann hierbei hinsichtlich einer möglichst genauen Annäherung der Exponentialfunktion an die tatsächlich auftretenden Druckmaxima bzw. Druckminima unter Verwendung eines Approximationsalgorithmus bestimmt werden.

Die Erfindung wird nachfolgend anhand eines in der Zeichnung dargestellten Ausführungebeispieles näher erläutert. In dieser zeigen Fig.1 eine Diagrammansicht eines Herzens mit einer Vorrichtung zum intermittierenden Okkludieren des Koronarsinus, Fig.2 eine graphische Darstellung des Koronarsinusdruckverlaufes und Fig.3 die obere Hüllkurve des Druckverlaufes.

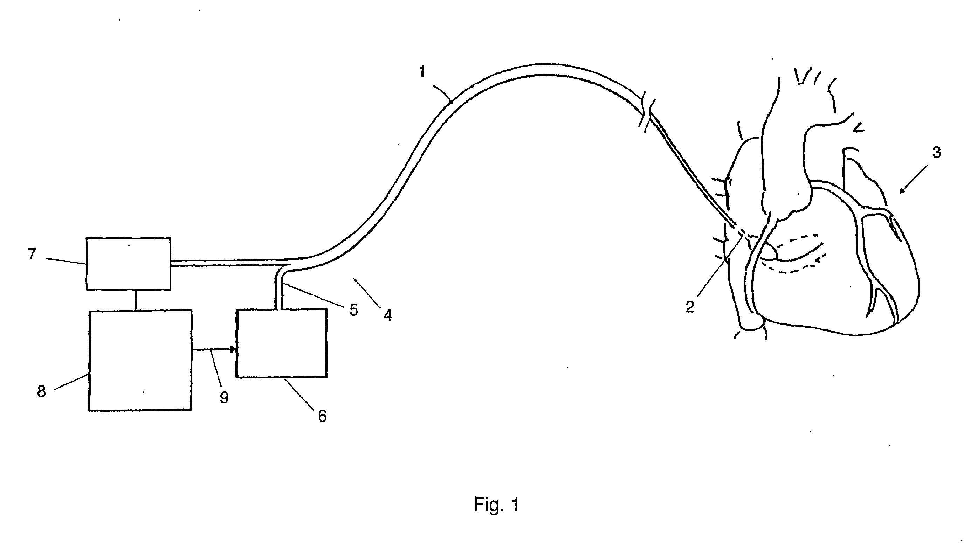

In Fig. 1 ist schematisch die Vorrichtung zum intermittierenden Okkludieren des Koronarsinus dargestellt, wobei ein Multilumen-Katheter 1, dessen distales Ende 2 in den Koronarsinus des Herzens 3 über das Atrium eingesetzt wird, ersichtlich ist. Das proximale Ende 4 des Katheters 1 hat ein Ballonaufblaslumen 5, das mit einer Pumpe 6 verbunden ist. Der am distalen Ende 2 des Katheters 1 herrschende Druck wird von einer Druckmesseinrichtung 7 erfasst, wobei die Druckmesseinrichtung auch einen Speicher für die ermittelten Messwerte aufweist. Die jeweiligen Druckmesswerte werden einer Steuerungseinrichtung 8 umfassend eine Recheneinheit zugeführt, welche Steuersignale über die Leitung 9 zum Starten und Anhalten der Pumpe 6 liefert.

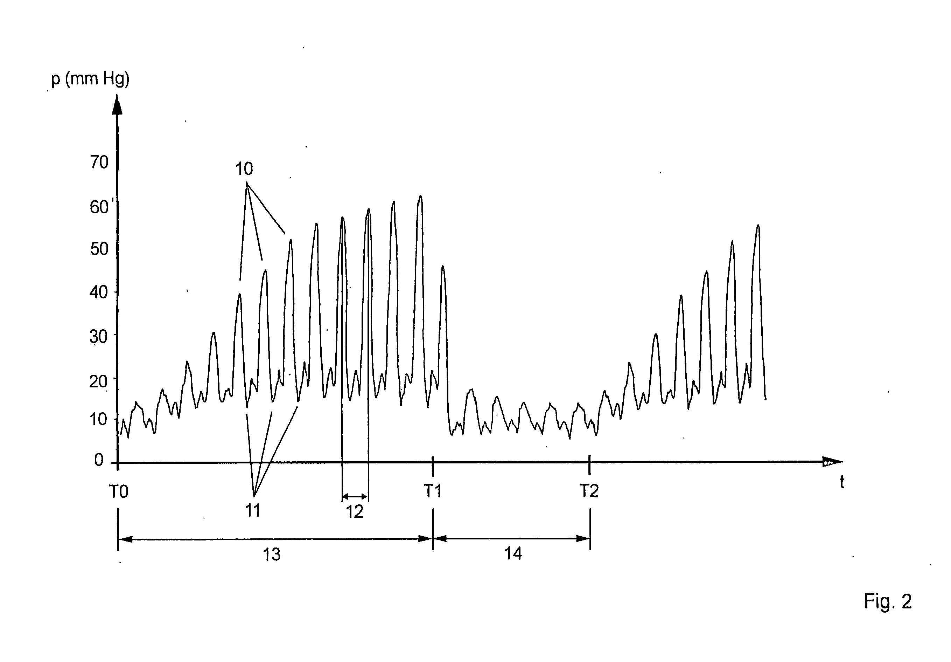

In Fig. 2 ist der von der Messeinrichtung 7 erfasste Druckverlauf dargestellt, wobei der Beginn der Okklusion mit T0, das Ende der Okklusion mit T1 und der Beginn der nächsten Okklusion mit T2 dargestellt ist. Es ist eine Reihe von systolischen Druckspitzen 10 und eine Reihe von diastolischen Tälern 11 ersichtlich. Die Pulsperiode 12 des Herzschlages wird durch die Zeit zwischen aufeinanderfolgenden Spitzen oder aufeinanderfolgenden Tälern dargestellt. Die Okklusionsphase ist mit 13 und die Releasephase mit 14 bezeichnet.

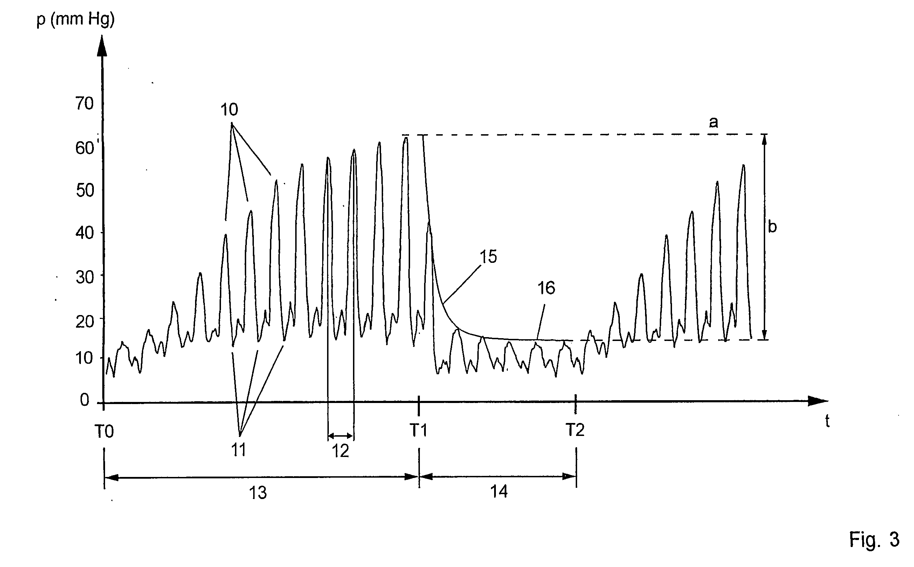

In Fig.3 ist für die Releasephase 14 die Hüllkurve 15 dargestellt, welche die Druckmaxima 10 verbindet und welche eine Exponentialfunktion darstellt. Die Exponentialfunktion 15 ist definiert durch:

$$p(t) = a + b \cdot e^{-t/c},$$

wobei

a    der systolische Druck im Koronarsinus vor der intermittierenden Okklusion,

b    die Differenz zwischen dem systolischen Druck am Ende der Okklusion und dem Druckniveau a,

c    eine Zeitkonstante und

t    die zeit gemessen vom Beginn der Releasephase darstellen.

Ausgehend von dieser Exponentialfunktion 15 kann das untere Plateau 16 des Druckverlaufs bestimmt werden und in der Folge auch die Zeit, welche benötigt wird, um den Plateauwert zu erreichen. Der tiefste Wert der Funktion p(t), d.h. der Plateauwert, wird ermittelt, in dem $t = \infty$ (unendlich) gesetzt wird. Für $t = \infty$ errechnet sich: $p(t) = a$. Dies bedeutet, dass die Druckkurve 15 sich dem Druckniveau asymptotisch nähert. Es kann daher nicht abgewartet werden bis die Druckkurve den Wert a erreicht, sondern es wird ein Vielfaches dieses Plateauwerts, beispielsweise das 1,01 fache des Plateauwerts, definiert, welcher den Zeitpunkt des Beginns der nächsten Okklusion bestimmt. Die Zeit, zu welcher das 1,01 fache des Plateauwerts erreicht wird, errechnet sich gemäß folgenden Gleichungen:

$$p(t_{1,01}) = 1,01 \cdot p(\infty),$$

$$1.01 \cdot a = a + b \cdot e^{-\frac{t_{1,01}}{c}},$$

$$t_{1,01} = c \cdot \ln \frac{b}{0,01 \cdot a}.$$

Entsprechend den Bedürfnissen können dieselben Rechenschritte für andere Vielfache des Plateauwerts vorgenommen werden.

Insgesamt erlaubt es die vorliegende Erfindung, den optimalen Zeitpunkt der Auslösung der Okklusion sehr genau zu bestimmen und somit die Dauer der Releasephase zu steuern. Dabei wird sichergestellt, dass die Okklusion erst nach Auftreten des Spitzenwertes der reaktiven Hyperämie wieder ausgelöst wird, wobei die Steuerung derart erfolgen kann, dass die Okklusion unmittelbar nach Auftreten diese Spitzenwerts ausgelöst wird, sodass die Dauer der Releasephase auf das notwenige Minimum reduziert werden kann. Durch Minimierung der Dauer der Releasephase wird das Verhältnis der Okklusionszeit zur Releasezeit erhöht und somit insgesamt über einen längeren Zeitraum die Okklusion durchgeführt, sodass über längere Zeit hindurch ein höherer Druck im Koronarsinus auftritt. Die möglichst lang andauernde Druckerhöhung führt hierbei vermehrt zur Freisetzung von gefäßbildenden Genen (VEGF-Gene, den vaskulären endothelialen Wachstumsfaktor kodierende Gene) kommt, sodass auch die Regeneration der Herzgefäße begünstigt wird.

**Patentansprüche**

1.   Vorrichtung zur intermittierenden Okklusion des Koronarsinus umfassend eine Okklusions-Einrichtung (2) zum abwechselnden Okkludieren des Koronarsinus und Aufheben der Okklusion, eine Druckmesseinrichtung (7) zur Erfassung des Drucks im Koronarsinus und eine Steuerungseinrichtung (8) zur Abgabe von Steuersignalen an die Okklusions-Einrichtung zur Steuerung des Zeitpunkts des Beginns und des Aufhebens der Okklusion, und einer mit der Steuerungseinrichtung Verbundenen Recheneinheit, **dadurch gekennzeichnet, dass** die Recheneinheit zur rechnerischen Abschätzung des Verlaufe des nach Aufhebung der Okklusion im Koronarsinus auftretenden Flüssigkeitsdrucks ausgebildet ist, wobei der Zeitpunkt des Beginns der nächsten Okklusion in Abhängigkeit vom geschätzten Druckverlauf bestimmt wird.

2. Vorrichtung nach Anspruch 1, **dadurch gekennzeichnet, dass** die Recheneinheit zur Abschätzung der nach Aufhebung der Okklusion im Koronarsinus während aufeinanderfolgender Herzschläge auftretenden Druckmaxima ausgebildet ist, wobei der Zeitpunkt des Beginns der nächsten Okklusion in Abhängigkeit von den geschätzten Druckmaxima bestimmt wird.

3. Vorrichtung nach Anspruch 1 oder 2, **dadurch gekennzeichnet, dass** die Recheneinheit zur Abschätzung der nach Aufhebung der Okklusion im Koronarsinus während aufeinanderfolgender Herzschläge auftretenden Druckminima ausgebildet ist, wobei der Zeitpunkt des Beginns der nächsten Okklusion in Abhängigkeit von den geschätzten Druckminima bestimmt wird.

4. Vorrichtung nach Anspruch 1 oder 2, **dadurch gekennzeichnet, dass** die Recheneinheit zur Abschätzung der nach Aufhebung der Okklusion im Koronarsinus während aufeinanderfolgender Herzschläge auftretenden Druckmaxima durch eine Exponentialfunktion ausgebildet ist, wobei der Zeitpunkt des Beginns der nächsten Okklusion in Abhängigkeit vom Plateauwert der Exponentialfunktion bestimmt wird.

5. Vorrichtung nach einem der Ansprüche 1 oder 3, **dadurch gekennzeichnet, dass** die Recheneinheit zur Abschätzung der nach Aufhebung der Okklusion im Koronarsinus während aufeinanderfolgender Herzschläge auftretenden Druckminima durch eine Exponentialfunktion ausgebildet ist, wobei der Zeitpunkt des Beginns der nächsten Okklusion in Abhängigkeit vom Plateauwert der Exponentialfunktion bestimmt wird.

6. Vorrichtung nach Anspruch 4 oder 5, **dadurch gekennzeichnet, dass** die Okklusion des Koronarsinus aufgehoben wird, wenn die Exponentialfunktion ein definiertes Vielfaches des prognostizierten Plateauwertes erreicht.

7. Vorrichtung nach Anspruch 4, 5 oder 6, **dadurch gekennzeichnet, dass** die Exponentialfunktion definiert ist durch:

$$p(t) = a + b \cdot e^{-t/c},$$

wobei

    a der systolische bzw. diastolische Druck im Koronarsinus vor der intermittierenden Okklusion,
    b die Differenz zwischen dem systolischen bzw. diastolischen Druck am Ende der Okklusion und dem Druck a,
    c eine Zeitkonstante

darstellen.

## Claims

1. A device for the intermittent occlusion of the coronary sinus including an occlusion device (2) for the alternate occlusion of the coronary sinus and release of the occlusion, a pressure measuring device (7) for pressure registration in the coronary sinus, as well as a control device (8) for delivering control signals to the occlusion device to control the point in time of the beginning and release of the occlusion, and an arithmetic unit connected with the control device, **characterized in that** the arithmetic unit is configured to calculationally estimate the curve of the fluid pressure occurring in the coronary sinus after the release of the occlusion, wherein the time of the beginning of the next occlusion is determined as a function of the estimated pressure curve.

2. A device according to claim 1, **characterized in that** the arithmetic unit is configured to estimate the pressure maxima occurring in the coronary sinus during consecutive heart beats after the release of the occlusion, wherein the point in time of the beginning of the next occlusion is determined as a function of the estimated pressure maxima.

3. A device according to claim 1 or 2, **characterized in that** the arithmetic unit is configured to estimate the pressure minima occurring in the coronary sinus during consecutive heart beats after the release of the occlusion, wherein the point in time of the beginning of the next occlusion is determined as a function of the estimated pressure minima.

4. A device according to claim 1 or 2, **characterized in that** the arithmetic unit is configured to estimate by an exponential function the pressure maxima occurring in the coronary sinus during consecutive heart beats after the release of the occlusion, wherein the point in time of the beginning of the next occlusion is determined as a function of the plateau value of said exponential function.

5. A device according to any one of claims 1 or 3, **characterized in that** the arithmetic unit is configured to estimate by an exponential function the pressure minima occurring in the coronary sinus during consecutive heart beats after the release of the occlusion, wherein the time of the beginning of the next occlusion is determined as a function of the plateau value of said exponential function.

6. A device according to claim 4 or 5, **characterized in that** the occlusion of the coronary sinus is released if the exponential function reaches a defined multiple of the forecast plateau value.

7. A method according to claim 4, 5 or 6, **characterized in that** the exponential function is defined by:

$$p(t) = a + b . e^{-t/c},$$

wherby

a being the systolic or diastolic pressure in the coronary sinus prior to the intermittent occlusion,
b being the difference between the systolic or diastolic pressure at the end of the occlusion and the pressure a,
c being a time constant.

**Revendications**

1. Dispositif d'occlusion intermittente du sinus coronaire, comprenant un dispositif d'occlusion (2) pour l'occlusion du sinus coronaire et la levée de l'occlusion en alternance, un dispositif de mesure de pression (7) pour détecter la pression dans le sinus coronaire, et un dispositif de commande (8) pour fournir des signaux de commande au dispositif d'occlusion en vue de commander les instants du début et de la levée de l'occlusion, et une unité de calcul reliée au dispositif de commande, **caractérisé en ce que** l'unité de calcul est réalisée pour estimer mathématiquement le gradient de la pression de liquide présente dans le sinus coronaire suite à la levée de l'occlusion, l'instant du début de l'occlusion suivante étant déterminé en fonction du gradient de pression estimé.

2. Dispositif selon la revendication 1, **caractérisé en ce que** l'unité de calcul est réalisée pour estimer les maxima de pression qui surviennent dans le sinus coronaire suite à la levée de l'occlusion pendant ces battements de coeur consécutifs, l'instant du début de l'occlusion suivante étant déterminé en fonction des maxima de pression estimés.

3. Dispositif selon la revendication 1 ou 2, **caractérisé en ce que** l'unité de calcul est réalisée pour estimer les minima de pression qui surviennent dans le sinus coronaire suite à la levée de l'occlusion pendant des battements de coeur consécutifs, l'instant du début de l'occlusion suivante étant déterminé en fonction des minima de pression estimés.

4. Dispositif selon la revendication 1 ou 2, **caractérisé en ce que** l'unité de calcul est réalisée pour estimer par une fonction exponentielle les maxima de pression qui surviennent dans le sinus coronaire suite à la levée de l'occlusion pendant des battements de coeur consécutifs, l'instant du début de l'occlusion suivante étant déterminé en fonction de la valeur de plateau de la fonction exponentielle.

5. Dispositif selon l'une quelconque des revendications 1 ou 3, **caractérisé en ce que** l'unité de calcul est réalisée pour estimer par une fonction exponentielle les minima de pression qui surviennent dans le sinus coronaire suite à la levée de l'occlusion pendant des battements de coeur consécutifs, l'instant du début de l'occlusion suivante étant déterminé en fonction de la valeur de plateau de la fonction exponentielle.

6. Dispositif selon la revendication 4 ou 5, **caractérisé en ce que** l'occlusion du sinus coronaire est levée lorsque la fonction exponentielle atteint un multiple défini de la valeur de plateau prédite.

7. Dispositif selon la revendication 4, 5 ou 6, **caractérisé en ce que** la fonction exponentielle est définie par :

$$p(t) = a + b \cdot e^{-t/c},$$

où

a est la pression systolique ou diastolique dans le sinus coronaire avant l'occlusion intermittente,
b est la différence entre la pression systolique ou diastolique à la fin de l'occlusion et la pression a,
c est une constante de temps.

Fig. 1

Fig. 2

EP 1 755 702 B1

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 230996 A2